# EUROPEAN PATENT APPLICATION

(11) **EP 4 417 388 A1**
(43) Date of publication of application: **21.08.2024**
(21) Application number: 23305218.2
(22) Date of filing: 20.02.2023
(51) Int. Cl.: B29B 17/02, A61F 13/00, A61L 15/16

(54) **METHOD FOR RECYCLING POST-CONSUMER ABSORBENT ARTICLE**

(71) Applicant: Bostik SA, 92700 Colombes (FR)
(72) Inventor: BELLINI, Clement, 60280 Venette (FR); LAFERTE, Olivier, 60280 Venette (FR)
(74) Representative: Arkema Patent

(57) **Abstract**

The present invention relates to a method for recovering plastic material(s) from a post-consumer disposable absorbent article comprising a topsheet, a backsheet, an absorbent core, a hot melt adhesive composition H, wherein at least one of the topsheet and the backsheet comprises at least one plastic material,
wherein the hot melt adhesive composition H comprises:
∘ at least one polymer P selected from the group consisting of polyolefins, thermoplastic block copolymers of a vinyl-based aromatic hydrocarbon and a conjugated diene compound, and mixtures thereof;
∘ at least one tackifier T comprising at least one ester group or carboxylic acid group;

the method being characterized in that it comprises:
- a step i) of providing a post-consumer disposable absorbent article,
- a step ii) of immersing the post-consumer disposable absorbent article in an alkaline aqueous solution in conditions sufficient to obtain debonding of plastic material(s),
- a step iii) of separation and recovering of plastic material(s) from the medium.

## Description

### TECHNICAL FIELD

The present invention relates to a method for recycling post-consumer absorbent article.

The present invention also relates to a method for recovering plastic materials from post-consumer absorbent articles.

### TECHNICAL BACKGROUND

Hundred of billions of dirty disposable absorbent articles (such as diapers) are disposed annually as solid wastes. Only a small part is recycled, mainly due to lack of technologies adapted for this kind of article.

The complexity of disposable absorbent articles arises from the consumer's need for a comfortable, effective, easy-to-use, and low-cost product. However, such system has made traditional recycling impossible to do efficiently and cost-effectively with current technologies. Consequently, post-consumer disposable diapers end up as solid waste piling up in landfill fields or are incinerated.

Use of either incineration or landfill disposal results in loss or destruction of the components of absorbent article rather than recycling some or all of the components to the same or other end-uses. The disposal problems are of ever increasing concern to environmental and governmental authorities.

More recently, there has been some attempts to recycle the used disposable absorbent articles. However, some methods typically use important quantity of hazardous extraction solvents. Other methods first comprise the shredding of disposable absorbent article into small pieces before washing processes, which do not allow recovering of good quality of each substrate of the disposable absorbent article, and demand additional purification steps.

There is a need for a new method of recycling used disposable absorbent article that remediate at least partly of at least one the mentioned drawbacks.

There is more particularly a need for efficient and economical processes for recycling used disposable absorbent articles into individual constituents without harming environment.

There is a need for efficient, easy to implement and economical processes for recycling used disposable absorbent articles.

### DESCRIPTION OF THE INVENTION

### A. Method for recovering

The present invention relates to a method for recovering plastic material(s) from a post-consumer disposable absorbent article comprising a topsheet, a backsheet, an absorbent core, a hot melt adhesive composition H, wherein at least one of the topsheet and the backsheet comprises at least one plastic material,
wherein the hot melt adhesive composition H comprises:
   ∘ at least one polymer P selected from the group consisting of polyolefins, thermoplastic block copolymers of a vinyl-based aromatic hydrocarbon and a conjugated diene compound, and mixtures thereof;
   ∘ at least one tackifier T comprising at least one ester group or carboxylic acid group;
the method being characterized in that it comprises:
   - a step i) of providing a post-consumer disposable absorbent article,
   - a step ii) of immersing the post-consumer disposable absorbent article in an alkaline aqueous solution in conditions sufficient to obtain debonding of plastic material(s),
   - a step iii) of separation and recovering of plastic material(s) from the medium.

### Post-consumer disposable absorbent article

"Absorbent article" is used herein to refer to consumer products whose primary function is to absorb and retain soils and wastes.

"Diaper" is used herein to refer to absorbent article generally worns by infant and incontinent persons about the lower torso.

The term "post-consumer article" concerns an article which has been used at lest one time by a consumer.

The term "disposable" is used herein to describe absorbent article which generally are not intended to be laundered or otherwise restored or reused as an absorbent article (e.g they are intended to be discarded after a single use).

The term "non-woven" refers herein to a material made from continuous (long) filaments (fibers) and/or discontinuous (short) filaments (fibers) by processes such as for example spundbonding, meltblowing, carding and the like. Non-woven do not have a woven or knitted filament pattern.

The disposable absorbent article may be a disposable diaper, adult incontinence article, sanitary napkins, medical dressing (such as wound care products), surgical pads, pet training pads. Preferably, the disposable absorbent article is a disposable diaper.

The disposable absorbent article comprises a topsheet, a backsheet, an absorbent core and a hot melt adhesive H. The disposable absorbent article may further comprise one or several hot melt adhesive(s) for example for bonding of elastic material, core material, etc...

Typically, disposable absorbent articles comprise a liquid permeable topsheet that faces the wearer's body, a liquid impermeable backsheet that faces the wearer's clothing (garment), an absorbent core interposed between the liquid permeable topsheet and the backsheet, and means to keep the core in fixed relation to the wearer's body.

The plastic material is preferably selected from the group consisting of: polyolefins (such as for example polyethylene, polypropylene, ...), polyesters (such as for example polyethylene terephthalate, polybutylene terephthalate), polyamides (such as for example 6-nylon, 6,6-nylon), poly(lactic acid), and mixtures thereof.

### Absorbent core

The function of the absorbent core is typically to absorb and retain exudates for a prolonged amount of time, minimize re-wet to keep the wearer dry and avoid soiling of clothes or bed sheets.

The majority of absorbent core typically comprise an absorbent material within a core wrap.

The absorbent core may comprise different absorbent materials including natural cellulose fibers (such as for example wood pulp, fibers, cotton or fluff), superabsorbent polymers (SAP), or mixtures thereof.

The superabsorbent polymers (SAP) may be selected from the group consisting of: modified natural gums, carboxymethyl cellulose, alkaline metal salts of acrylic polymers, polyacrylamide, polyvinyl alcohol, polyvinyl ether (co)polymers, hydroxyalkyl cellulose (co)polymers, polyvinyl sulfonic acid (co)polymers, polyacrylic acid (co)polymers, and mixtures thereof.

Preferably, the absorbent core comprises superabsorbent polymer (SAP).

Examples of superabsorbent polymers are disclosed in WO2009/155265 or WO2009/155264.

Fluid distributing channels extending longitudinally may be used. The channels can distribute an insulating fluid quickly along a greater area of the absorbent core thus improving fluid acquisition and optimizing absorbent material usage. Channels may also be used to facilitate the folding of the absorbent core in a pre-determined fashion, thus improving the anatomical conformity of the article.

Core wrap may typically comprise one or two layers of non-woven plastic material such as PP (polypropylene) or PE (polyethylene).

### Topsheet

The liquid permeable topsheet advantageously allows liquids (e.g. menses and/or urine) to readily penetrate through its thickness.

The topsheet may comprise woven and non-woven materials, porous foams, reticulated foams, synthetic films, and mixtures thereof.

The non-woven material may comprise natural fibers (for example wood fibers, cotton fibers, bamboo fibers, Lyocell fibers, ...), synthetic fibers (for example polymeric fibers such as polyesters, polyolefins, ethylene vinyl acetate polymers, ethylene acrylic acid polymers, and mixture thereof), and mixtures thereof.

According to the invention, the (post-consumer) disposable article is such that at least one of the topsheet and the backsheet comprise at least one plastic material.

Preferably, the topsheet comprises at least one plastic material.

The topsheet may comprise one or several different plastic materials.

The different plastic materials may be selected from different type of plastics for example the first being a polyolefin and the second being a polyester, or they can be from the same type : for example polyolefins but the first being a polyethylene and the second a polypropylene.

The plastic material(s) in the topsheet may be identical or different from the plastic material(s) of the backsheet.

Plastic material typically includes nonwoven material, plastic films, rubbers and the like.

The plastic material is preferably selected from the group consisting of: polyolefins (such as for example polyethylene, polypropylene, ...), polyesters (such as for example polyethylene terephthalate, polybutylene terephthalate), polyamides (such as for example 6-nylon, 6,6-nylon), poly(lactic acid), and mixtures thereof.

The topsheet preferably comprises a non-woven material, said non-woven material preferably comprising plastic material. Even more preferably, the topsheet comprises a non-woven material comprising polypropylene (more particularly fibers of polypropylene).

The topsheet may have a basis weight of from 10 to 28 gsm, in particular 12 to 18 gsm, but other basis weights are possible.

### Backsheet

The backsheet advantageously prevents the exudates absorbed and contained in the absorbent core from wetting articles which contact the absorbent article.

The backsheet is typically impermeable to liquids (such as example, urine).

The backsheet may comprise woven or non-woven material, synthetic films, or composite material of nonwoven materials and synthetic films.

The non-woven material may comprise natural fibers (for example wood or cotton fibers), synthetic fibers (for example polymeric fibers such as polyesters, polyolefins, ethylene vinyl acetate polymers, ethylene acrylic acid polymers, and mixture thereof), and mixtures thereof.

Preferably, the backsheet comprises at least one plastic material.

The backsheet may comprise one or several different plastic materials

The plastic material in the backsheet may be identical or different from the plastic material(s) of the topsheet.

The different plastic materials may be selected from different type of plastics for example the first being a polyolefin and the second being a polyester, or they can be from the same type: for example polyolefins but the first being a polyethylene and the second a polypropylene.

Plastic material typically includes non-woven material, plastic films, rubbers and the like.

The plastic material is preferably selected from the group consisting of: polyolefins (such as for example polyethylene, polypropylene, ...), polyesters (such as for example polyethylene terephthalate, polybutylene terephthalate), polyamides (such as for example 6-nylon, 6,6-nylon), poly(lactic acid), and mixtures thereof.

The backsheet preferably comprises a plastic film comprising a plastic material, and more particularly a plastic material selected from polyethylenes.

The backsheet is preferably joined to the topsheet, the absorbent core, or any other elements of the disposable absorbent article by any attachments means known in the art. Suitable attachments means are for example uniform continuous layer of adhesive, a patterned layer of adhesive, or an array of separate lines, spirals or spots of adhesive.

The topsheet and the backsheet are preferably joined via the hot melt adhesive composition H as defined herein.

The backsheet may have a basis weight of from 10 to 28 gsm, in particular 15 to 28 gsm, but other basis weights are possible.

### Additional layers

The disposable absorbent article may also comprise additional layers, such as an acquisition layer, a distribution layer, and mixtures thereof.

The function of an acquisition layer is typically to quickly draw the fluid away from the topsheet to keep the skin dry.

The acquisition layer may be typically disposed between the topsheet and the absorbent core.

Typical examples of acquisition layers are air-through bonded carded web, with a basis weight ranging from 10 gsm to 60 gsm are known in the art.

The additional layer(s) may be of any kind such as for example nonwoven, a woven material or loose fibers.

The additional layer(s) may comprise one or several plastic materials, which may be identical or different from the plastic materials of other layers.

### Hot melt adhesive H

By *"hot melt"* is meant that the adhesive composition requires to be heated at least at 100°C, preferably at least at 140°C, to be applied on a substrate. The hot melt adhesive composition is thus solid at 23°C.

The hot melt adhesive composition H comprises:
∘ at least one polymer P selected from the group consisting of polyolefins, thermoplastic block copolymers of a vinyl-based aromatic hydrocarbon and a conjugated diene compound, and mixtures thereof;
∘ at least one tackifier T comprising at least one ester group or carboxylic acid group.

### Polyolefins

The polyolefins may be homopolymers or copolymers.

The polyolefins are well known in the field of adhesives. They may be prepared using a metallocene catalyst or Ziegler-Natta catalyst.

Olefins are unsaturated hydrocarbons and the most typical monomers used in polyolefins are ethylene and alpha-olefins containing up to ten carbon atoms. Principal olefin monomers include ethylene, propylene, butene-1,4-methylpentene, hexene, octene and combinations thereof. Polyolefins include ethylene polymers, propylene polymers, and combinations thereof including combinations with other alpha-olefins.

Propylene-based co-polymers may comprise at least 50 percent of propylene monomers by weight of the copolymer.

Ethylene-based co-polymers may comprise at least 50 percent of ethylene monomers by weight of the copolymer.

The remaining monomers may be other alpha olefin monomers may be present in the co-polymers, for example 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof.

The exact monomer distribution is typically published by the supplier, but can also be determined by a suitable method, such as nuclear magnetic resonance or infrared spectroscopies.

Suitable metallocene-catalyzed propylene-ethylene copolymers are commercially available from Clariant under the polymer range Licocene^{®}, with a broad range of properties such as molecular weight, viscosity, crystallinity, etc...

Other suitable commercially available propylene-ethylene copolymers are available as Vistamaxx^{®} copolymers from Exxon. Vistamaxx typically have a higher molecular weight than Licocene copolymers. Metallocene-catalyzed propylene homopolymers are available from Idemitsu Kosan Co., Ltd under the tradename L-MODU^{™}, in particular L-Modu S-410.

### Thermoplastic block copolymers

The hot melt adhesive composition H preferably comprises one or several thermoplastic block copolymers of a vinyl-based aromatic hydrocarbon and a conjugated diene compound.

The "vinyl-based aromatic hydrocarbon" means an aromatic hydrocarbon compound having a vinyl group. Specific examples thereof include styrene, o-methylstyrene, p-methylstyrene, p-tert-butylstyrene, 1,3-dimethylstyrene, a-methylstyrene, vinylnaphthalene and vinylanthracene. Particularly, styrene is preferred. These vinyl-based aromatic hydrocarbons may be used alone or in combination.

The "conjugated diene compound" means a diolefin compound having at least a pair of conjugated double bonds. Specific examples of the "conjugated diene compound" may include 1,3-butadiene, 2-methyl-1,3-butadiene (or isoprene), 2,3-dimethyl-1,3-butadiene, 1,3-pentadiene and 1,3-hexadiene. 1,3-butadiene and 2-methyl-1,3-butadiene are particularly preferred. These conjugated diene compounds may be used alone or in combination.

The thermoplastic block copolymer may be either a non-hydrogenated or hydrogenated thermoplastic block copolymer.

Specific examples of the "non-hydrogenated thermoplastic block copolymer" may include block copolymers in which block(s) based on the conjugated diene compound are not hydrogenated. Specific examples of the "hydrogenated thermoplastic block copolymer" include block copolymers in which all or a part of the blocks based on the conjugated diene compound are hydrogenated.

Preferably, the thermoplastic block copolymer is a styrene-based block copolymer. The styrene-based block copolymer means a polymer having at least one styrene block. The styrene block means a segment comprising styrene as a main monomer, and it is preferred that the segment is substantially composed of styrene only.

Specific examples of the "non-hydrogenated thermoplastic block copolymer" include a styrene-isoprene-styrene block copolymer (also referred to as "SIS") and a styrene-butadiene-styrene block copolymer (also referred to as "SBS"). Specific examples of the "hydrogenated thermoplastic block copolymer (A)" include a hydrogenated styrene-isoprene-styrene block copolymer (also referred to as "SEPS"), a hydrogenated styrene-butadiene-styrene block copolymer (also referred to as "SEBS"), and styrene-butylene/butadiene-styrene block copolymer (also referred to as "SBBS").

The thermoplastic block copolymer is preferably chosen from a linear diblock copolymer having an AB structure (preferably a copolymer comprising one terminal styrene block and one block formed from at least one type of monomers other than styrene monomers), a linear triblock copolymer having an ABA structure (preferably a copolymer comprising two terminal styrene blocks and a mid-chain block formed from at least one type of monomers other than styrene monomers), a radial block copolymer having the (AB)ₙY structure, and mixtures of such copolymers.

A represents a non-elastomeric polystyrene block.

B represents an elastomeric block polymer. B may notably be a polydiene, e.g. polybutadiene and/or polyisoprene block.

Y represents a multivalent compound.

Finally, n is an integer which is equal to at least 3.

Preferably, the thermoplastic block copolymer is chosen from the group consisting of:
- styrene-butadiene-styrene copolymer (SBS),
- styrene-butadiene diblock (SB),
- styrene-isoprene-styrene copolymer (SIS),
- styrene-isoprene diblock (SI),
- styrene-ethylene-butylene-styrene copolymer (SEBS),
- styrene-propylene-butylene-styrene copolymer (SPBS),
- styrene-butadiene-butylene-styrene copolymer (SBBS),
- styrene-ethylene-propylene-styrene copolymer (SEPS),
- styrene-ethylene-ethylene-propylene-styrene copolymer (SEEPS),
- styrene-isoprene-butadiene-styrene copolymer (SIBS),
- styrene-isoprene-propylene-styrene copolymer (SIPS),
- styrene-farnesene copolymer (SFC),
- and any mixture thereof.

The thermoplastic block copolymer preferably comprises a styrene content ranging 10% to 60% by mass.

Useful commercial thermoplastic block copolymers include KRATON D and G ^{®} series from Kraton Polymers, EUROPRENE Sol T ^{®} series from Versalis (Eni group), SOLPRENE ^{®} series from Dynasol Elastomers, SINOPEC ^{®} series from SINOPEC and Taipol ^{®} and Vector^{®} series from TSRC Corporation.

Other useful examples include:
- Sinopec^{®} YH-1126, a linear SIS triblock copolymer with a styrene content of 16 %, and a diblock content of 50 %;
- Sinopec^{®} YH-1209, a linear SIS triblock copolymer with a styrene content of 30 %, and a diblock content of less than 1 %;
- Kraton^{®} D1152, a mixture of linear SBS triblock and SB diblock copolymers, with a styrene content of 29.5 % by weight relative to the total weight of the mixture, an average molecular weight of around 122 000 g/mol, a melt flow index or MFI (measured according to ISO1133) of 8.5 grams/ 10 minutes at 200°C under a load of 5 kilograms, and a SB diblock content of around 17 % by weight relative to the total weight of the mixture.
- Kraton^{®} D1161, a mixture of linear SIS triblock and SI diblock copolymers, with a styrene content of 15 % by weight relative to the total weight of the mixture, an MFI (measured according to ISO1133) of 9 g/10 min at 200°C under a load of 5 kg, an average molecular weight of around 220 000 g/mol, and a SI diblock content of around 19 % by weight relative to the total weight of the mixture.
- Taipol^{®} SBS 4202 from TSRC Corporation, a linear SBS triblock copolymer with a styrene content of 40 % by weight relative to the total weight of the triblock copolymer, an MFI (measured according to ASTM D1238) of 3 to 10 g/10 min at 190°C under a load of 5 kg, an average molecular weight of around 102 400 g/mol.
- Vector^{®} 4411 from TSRC Corporation, a linear SIS triblock copolymer with a styrene content of 44 % by weight relative to the total weight of the triblock copolymer, an MFI (measured according to ASTM D1238) of 40 g/10 min at 200°C under a load of 5 kg, an average molecular weight of around 106 000 g/mol.

More preferably, the hot melt adhesive composition H comprises
- a mixture of a linear triblock copolymer having an ABA structure and a linear diblock copolymer having an AB structure, as defined above, more preferably a mixture of SIS and SI copolymers; or
- a linear triblock copolymer having a ABA structure, and more preferably SIS copolymer.

The hot melt adhesive composition H may comprise from 1 to 50 %, preferably from 5 to 40 %, more preferably from 10% to 40% by weight of the at least one polymer P (or of the mixture of polymers P in case a plurality of polymers P are present in the hot melt adhesive composition) relative to the total weight of the composition.

### Tackifier T

As used herein, the terms "tackifier" and "tackifying resin" are used interchangeably.

The hot melt adhesive composition H comprises at least one tackifier T comprising at least one ester group or carboxylic acid group.

The tackifier T is preferably selected from the group consisting of:
- natural and modified rosins such as, for example, gum rosins, wood rosins, tall-oil rosins, distilled rosins, (partially of fully) hydrogenated rosins, dimerized rosins and polymerized rosins;
- ester of natural and modified rosins,
- and mixtures thereof.

The ester of natural and modified rosins (including hydrogenated and non-hydrogenated rosins) are preferably glycol, glycerol or pentaerythritol esters of natural and modified rosins, such as, for example, the glycerol esters of pale wood rosin, the glycerol esters of (partially of fully) hydrogenated rosin, the glycerol esters of polymerized rosin, the pentaerythritol esters of pale wood rosin, the pentaerythritol esters of (partially of fully) hydrogenated rosin, the pentaerythritol esters of tall oil rosin and the phenolic modified pentaerythritol esters of rosin.

The tackifier T may have a Ring and Ball softening point from 20°C to 150°C, preferably from 50°C to 150°C. The softening point may be measured by using the ASTM E28 standard.

As examples of commercially available tackifier resins mention may be made of: unmodified natural tall oil rosins from Arizona Chemical Company sold under the trade names Sylvaros^{®} (85, 90 and NCY), the partially hydrogenated rosin from Eastman sold under the trade name Foralyn^{®} E; the fully hydrogenated rosin from Eastman sold under the trade name Foral^{®} AX-E; the fully hydrogenated rosin from DRT sold under the trade name Foral^{®}DX; the fully hydrogenated rosin ester from DRT sold under the trade name Foral^{®} 105, Sylvalite^{®} RE 100L from Kraton (a pentaerythritol based tall-oil rosin ester), Sylvalite^{®} RE 85L sold by Kraton (a glycerol ester of tall oil rosin), Sylvalite^{®}9000 sold by Kraton (tall oil rosin ester having R&B softening point of 103°C).

The hot melt adhesive composition H preferably comprises from 0.5% to 70 %, more preferably from 20% to 70%, even more preferably from 20% to 50% by weight of the tackifier T (or of the mixture of tackifier T in case a plurality of tackifier resins T are present in the hot melt adhesive composition H) relative to the total weight of the said hot melt.

### Other tackifiers T'

The hot melt adhesive composition H may further comprise a tackifier T' different from tackifier T as defined above.

The tackifier T' is preferably selected from the group consisting of:
- Aliphatic and cycloaliphatic petroleum hydrocarbon resins and the hydrogenated derivatives thereof;
- Aromatic petroleum hydrocarbon resins and the hydrogenated derivatives thereof;
- Aromatic modified aliphatic or cycloaliphatic petroleum hydrocarbon resins and the hydrogenated derivatives thereof;
- polyterpene homopolymers which typically may result from the polymerization of one or several terpene hydrocarbons, such as for example the monoterpenes known as pinene, for example in the presence of Friedel-Crafts catalysts;
- copolymers of terpene with a diene monomer (for example styrene, methylstyrene, etc);
- phenolic-modified terpene resins such as, for example those resulting from the condensation, in an acidic medium, of a terpene and a phenol;
- and mixtures thereof.

The aliphatic petroleum hydrocarbon resins result typically from the polymerization of monomers consisting primarily of aliphatic olefins and di-olefins. The aliphatic petroleum hydrocarbon resins, and the hydrogenated derivatives thereof, may be based on C5 olefins such as for example cis/trans 1,3-pentadienes, 2-methyl-2-butene.

Examples of aliphatic petroleum hydrocarbon resins (and hydrogenated derivatives) are those commercially available under the tradenames Eastotac^{®} H100W (resin C5 fully hydrogenated) from Eastman Chemical Company, or Eastotac^{®} H100W (resin C5 fully hydrogenated).

The cycloaliphatic petroleum hydrocarbon resins result typically from the polymerization of monomers consisting primarily of cycloaliphatic unsaturated hydrocabons. It may be based on dicyclopentadiene or its derivatives (methyldicyclopentadiene, dimethyldicyclopentadiene, ...).

Aromatic modified aliphatic or cycloaliphatic petroleum hydrocarbon resins may be obtained from copolymerization of aliphatic olefins (such as for example C5) or cycloaliphatic, with aromatic olefins (such as for example C9) (optionally followed by partial or total hydrogenation).

Commercial tackifiers T' are for example polyterpene tackifiers from Arizona Chemical company sold under the trade names Sylvagum^{®} TR and Sylvares^{®} TR series (7115, 7125, A25L, B115, M1115); the polyterpene from DRT sold under the trade name DERCOLYTEOM105, resulting from the polymerization of α-pinene and β-pinene and having a Ring and Ball softening point of 105°C, the terpene phenol resins from the Arizona Chemical Company sold under the trade names Sylvares^{®} TP (96, 2040, 300, 7042, 2019).

The hot melt adhesive composition H preferably comprises from 10% to 70 %, more preferably from 10% to 40%, even more preferably from 15% to 40% by weight of the tackifier T' (or of the mixture of tackifier T' in case a plurality of tackifier resins T' are present in the hot melt adhesive composition H) relative to the total weight of the said hot melt.

Preferably, the hot met adhesive composition H does not comprise α-methylstyrene-based tackifying resin. Such tackifying resins are typically odorous and expensive.

Examples of α-methylstyrene-based tackifying resin are typically Kistalex 3085, Kristalex 1120 commercialized by Eastman Chemical.

### Plasticizers

Preferably, the hot melt adhesive composition comprises at least one plasticizer.

The plasticizer may be selected from the group consisting of naphthenic and paraffinic oils, liquid olefin polymers, plasticizers comprising at least one ester group or carboxylic acid group, and mixtures thereof.

Naphthenic oils and paraffinic oils are petroleum-based oils which consist in a mixture of naphthenic hydrocarbons (e.g. aliphatic, saturated or unsaturated, C4 to C7-member hydrocarbon rings; preferably aliphatic, saturated or unsaturated, C4 to C6-member rings, including cycloalkanes such as cyclopentane, cyclohexane, cycloheptane), paraffinic hydrocarbons (saturated, linear or branched, alkanes) and aromatic hydrocarbons (aromatic hydrocarbon rings, which may be monocyclic or polycyclic, and preferably aromatic C6-member hydrocarbon rings).

The classification of naphthenic and paraffinic oil is made based on the amount of each type of hydrocarbons in the oil. Typically, paraffinic oils have a paraffinic hydrocarbons content of at least about 50% by weight; naphthenic oils have a naphthenic hydrocarbons content from about 30% to about 50% by weight, relative to the total weight of the plasticizer.

Examples of naphtenic oils are Nyflex^{®} 223 and Nyflex^{®} 222B commercialized by Nynas, or Primol^{®} 352 and 382 from Exxon Mobil.

The liquid olefin polymers may be selected from the group consisting of polypropylene, polybutene, hydrogenated polyisopropene, hydrogenated butadiene, or the like having average molecular weights between about 100 and about 140,000 g/mol, polyfarnesene preferably with average molecular weights higher or equal to 135 000 g/mol.

The plasticizers comprising at least one ester group or acid carboxylic group are preferably selected from the group consisting of: vegetable oils, animal oils, and derivatives thereof.

A vegetable oil is a composition comprising triple esters of fatty acids and of glycerol (also called *"triglycerides"*)*.*

The vegetable oil may be selected from the group consisting of castor oil, sunflower oil, coconut oil, corn oil, canola oil, olive oil, palm oil, cottonseed oil, rapeseed oil, safflower oil, soybean oil, sesame oil, olive oil, almond oil, avocado oil, hemp oil, linseed oil, and mixtures thereof.

The fatty acid may be a hydrogenated or non-hydrogenated fatty acid.

The fatty acid may be chosen from myristic acid, palmitic acid, stearic acid, oleic acid, linoleic acid, and ricinoleic acid.

The derivatives of vegetable oils may be esterified fatty acids, fatty acid oligomers/polymers, and mixtures thereof.

Esterified fatty acids may result from the transesterification reaction between a mono-alcohol (preferably with low number molecular weight) and the triglyceride. In this case, the mono-alcohol is preferably chosen from methanol, ethanol, propanol, isopropanol, butanol, and 2-ethylhexanol.

An example of an esterified fatty acid resulting from the transesterification reaction between a mono-alcohol and the triglyceride may be the commercially available RADIA 7916 commercialized by OLEON.

The esterified fatty acid may also result from a transesterification reaction between a polyol and the triglyceride. In this case, the polyol may be chosen from glycerol and pentaerythritol. An example of an esterified fatty acid resulting from a transesterification reaction between a polyol and the triglyceride may be the commercially available PIONIERO TP130S from H&R which is a hydrogenated esterified soybean oil and the commercially available RADIA 7176 (Pentaerythritol Tetrastearate) commercialized by OLEON.

Fatty acid oligomers/polymers may be result from:
- the polycondensation of one fatty acid or a mixture of fatty acids;
- the polycondensation of vegetable oil with one fatty acid.

Preferably, the fatty acid oligomers/polymers are ricinoleic acid oligomers/polymers obtained by polycondensation of ricinoleic acid.

Examples of fatty acid oligomers/polymers resulting from the polycondensation of vegetable oil with one fatty acid are Crodabond SA commercialized by CRODA (acid value = 1 mg KOH/g).

Preferably, the plasticizer has an acid value lower or equal to 10 mg KOH/g.

Preferably, the plasticizer is selected from fatty acid oligomers/polymers.

The hot melt adhesive composition H preferably comprises from 0% to 50 %, more preferably from 1% to 40%, even more preferably from 5% to 20% by weight of plasticizer(s) relative to the total weight of the said hot melt.

### Waxes

The hot melt adhesive composition may further comprise a wax.

The wax may be present in the hot melt adhesive composition H at a content from 0% to 40 % and preferably from 5% to 30% by weight relative to the total weight of the hot melt adhesive composition.

Waxes typically include microcrystalline waxes, paraffin waxes, polyethylene waxes, polypropylene waxes, by-product polyethylene waxes, and Fischer-Tropsch waxes.

Examples of Fischer-Tropsch waxes are available from Sasol-SA under commercial name PARAFLINT^{®} H-1, H-4 and H-8, and from Shell under commercial name GTL Sarawax.

### Additives

The hot melt adhesive composition of the invention may optionally include one or several additives. Such additives may include waxes, antioxidants, pigments, brightening agents, colorants, fluorescing agents, dyestuffs, fillers, flow and leveling agents, wetting agents, surfactants, antifoaming agents, rheology modifiers, emulsifiers, humectants, gelling agent, colorants, other surface modifying agents, fragrances, and permeation enhancers.

Additives may be incorporated in minor amounts, for example up to about 10 %, preferably up to 5 %, by weight, into the hot melt adhesive composition H.

Useful antioxidants may include for example, pentaerythritol tetrakis[3,(3,5-di-tert-butyl-4-hydroxyphenyl)propionate], 2,2'-methylene bis(4-methyl-6-tert-butylphenol), phosphites including, e.g., tris-(p-nonylphenyl)-phosphite (TNPP) and bis(2,4-di-tert-butylphenyl)4,4'-diphenylene-diphosphonite, di-stearyl-3,3'-thiodipropionate (DSTDP), and combinations thereof.

Useful antioxidants are commercially available under a variety of trade names including, e.g., the IRGANOX series of trade names including IRGANOX 1010, IRGANOX 565, and IRGANOX 1076 hindered phenolic antioxidants and IRGAFOS 168 phosphite antioxidant, all of which are available from BASF Corporation, and ETHYL 702 4,4'-methylene bis(2,6-di-tert-butylphenol).

### Preparation of the hot melt adhesive

The hot melt adhesive composition H may be prepared by mixing the polymer P with the tackifier resin, the optional plasticizer and any other additional compounds and then heating this mixture at a temperature from 100 to 180 °C and for a duration from 1 to 8 hours.

Alternatively, the hot melt adhesive composition H may be prepared by heating a mixture formed from the polymer P, the optional plasticizer and any other additional compounds at a temperature from 100 to 180 °C and for a duration from 1 to 8 hours and then adding the tackifier resin.

The composition may then be cooled at a temperature from 20 to 30°C, and preferably at a temperature around 23°C (ambient temperature).

The hot melt adhesive composition H is preferably not a hot melt pressure-sensitive adhesive.

The hot met adhesive composition H preferably comprises:
∘ from 5% to 40% by weight of thermoplastic block copolymers of a vinyl-based aromatic hydrocarbon and a conjugated diene compound, and mixtures thereof;
∘ from 20% to 70% by weight of tackifier(s) T comprising at least one ester group or carboxylic acid group;
∘ from 1 % to 40% by weight of plasticizer(s) selected from vegetable oils, animal oils, and derivatives thereof.

The hot melt adhesive composition H has preferably a viscosity at 149°C ranging from 200 to 15 000 mPa.s, more preferably ranging from 300 to 13 000 mPa.s, and even more preferably ranging from 300 to 5 000 mPa.s. This viscosity is measured by using the ASTM method D-3236.

The hot melt adhesive composition H has preferably a Ring and Ball softening point from 50°C to 120°C, preferably from 65°C to 100°C. The softening point may be measured by using the ASTM E28 standard.

The hot melt adhesive composition H preferably exhibits an average initial dry peel strength higher than 2.0 N/25 mm, more preferably higher than 2.0 N/25 mm, even more preferably higher than 2.5 N/25 mm with an adhesive coating weight of 5 g/m². The average initial dry peel strength is measured according to the method entitled "Initial dry peel measured at 23 °C" described below. The "initial dry peel strength" is typically the dry peel strength of the hot melt adhesive composition before washing conditions of the method of recycling.

The hot melt adhesive composition H preferably exhibits an average initial wet peel strength higher than 2.0 N/25 mm, more preferably higher than 2.0 N/25 mm, even more preferably higher than 2.5 N/25 mm with an adhesive coating weight of 5 g/m². The average wet peel strength is measured according to the method entitled "Initial wet peel measured at 23°C" described below. The "initial wet peel strength" is typically the wet peel strength of the hot melt adhesive composition before washing conditions of the method of recycling.

### Method - steps

The present invention relates to a method for recovering plastic material(s) from a post-consumer disposable absorbent article comprising a topsheet, a backsheet, an absorbent core, a hot melt adhesive H as defined herein, wherein at least one of the topsheet and the backsheet comprises at least one plastic material,
the method being characterized in that it comprises:
- a step i) of providing a post-consumer disposable absorbent article,
- a step ii) of immersing the post-consumer disposable absorbent article in an alkaline aqueous solution in conditions sufficient to obtain debonding of plastic material(s),
- a step iii) of separation and recovering of plastic material(s) from the medium.

### Step ii)

The post-consumer disposable absorbent article of step ii) has advantageously not been submitted to a shredding step/breaking up step, and is used as such (not broken up into fragments).

The alkaline aqueous solution used herein has a pH greater than 7.

The alkaline aqueous solution is preferably an aqueous solution of sodium hydroxide, potassium hydroxide, lithium hydroxide, and mixtures thereof.

The concentration of hydroxide ions in the alkaline solution may range from 1 wt% to 10 wt%, preferably from 1 wt% to 5 w%.

More preferably, the alkaline aqueous solution is an aqueous solution of sodium hydroxide.

The pH of the alkaline aqueous solution preferably ranges from 8 to 14, more preferably from 10 to 14.

The pH of the alkaline aqueous solution may be measured according to known methods such as with pH-meter.

The conditions sufficient to obtain debonding of plastic material(s) are preferably:
- at a temperature lower than the softening temperature of the hot melt adhesive composition H,
- for a time ranging from 10 second to 24 hours, preferably 10 seconds to 6 hours.

More preferably, the conditions sufficient to obtain debonding of plastic material(s) are:
- at a temperature ranging from 23°C to 80°C,
- for a time ranging from 10 seconds to 2 hours.

Even more preferably, the conditions sufficient to obtain debonding of plastic material(s) are:
- at a temperature ranging from 30°C to 80°C,
- for a time ranging from 10 seconds to 15 minutes.

Step ii) is preferably carried out under mixing, for example with a mixer or mechanical agitator.

Step ii) advantageously allows debonding of plastic material(s) until complete separation (for example without additional mechanical peel).

Step ii) advantageously allows the debonding of plastic materials from other materials present in the post-consumer disposable absorbent article (such as for example cotton, natural fiber, fluff...), more particularly until complete separation of plastic materials from said other materials.

The adhesive properties such as the adhesion force of the adhesive layer resulting from the hot melt adhesive composition H advantageously diminishes during step ii). The peel adhesion may decrease by at least 80% or even at least 90%, and preferably at least 99% when the post-consumer disposable absorbent article is submitted to step ii).

More preferably, when the post-consumer disposable absorbent article comprises at least two different plastic materials, step ii) advantageously allows debonding of said two different plastic materials from each other. These at least two different plastic materials may be comprised in the same layer, such as for example in the topsheet, or may be comprised in separate layer such as for example one plastic material in the topsheet and one different plastic material in the backsheet, or each topsheet and backsheet can comprises two or more different plastic materials.

During step ii), the hot melt adhesive composition H present in the (post-consumer) disposable absorbent article (more particularly as an adhesive layer), advantageously loses its adhesion under the conditions to some degree enabling debonding of the plastic materials in contact with said hot melt adhesive composition H/adhesive layer.

Preferably, less than 20 wt% of the hot melt adhesive composition H dissolves in the alkaline aqueous solution, even more preferably less than 10 wt%, and even more advantageously less than 1 wt%. Thus, the adhesive residue in the medium resulting from step ii) is advantageously lower than 20wt%, preferably lower than 10 wt%, even more preferably lower than 1wt%. This advantageously allows avoiding additional depollution treatment of the washing solutions.

Advantageously, the hot met adhesive composition H stays mainly on the debonded plastic materials. More than 80wt% of the hot melt adhesive composition H preferably stays on the debonded plastic materials.

### Step iii)

The method according to the invention comprises a step iii) of separation and recovering of plastic material(s) from the medium.

The medium of step iii) is preferably the alkaline aqueous solution obtained after step ii). It may comprise any layer components that typically dissolve in alkaline aqueous solution, and/or any contaminants. The medium advantageously comprises less than 20 wt% of hot melt adhesive composition H, more preferably less than 10 wt% and even more preferably less than 1 wt%.

The separation step iii) may be carried out by appropriate mechanicals means, such as for example by filtration, by sedimentation, by decantation, by centrifugation.

In a preferred embodiment, at least one of the topsheet and the backsheet comprises at least two different plastic materials. According to this embodiment, the step iii) may allow:
- separation and recovering of a mixture of plastic materials from the medium (an additional step of sorting of plastic materials may be performed afterwards based on the difference of chemical nature); or
- separation and recovering of individual plastic material in isolation from the medium (avoiding mixtures of different plastic materials). For example in that case, if the topsheet comprises polypropylene as a first plastic material and the backsheet comprises a polyethylene as a second plastic material, then step iii) advantageously allows the sorting and the recovering of individual polypropylene in isolation, and another recovering of individual polyethylene in isolation. This separation may be done by gravimetric technique (depending of the density of each plastic material).

Preferably, the present invention relates to a method for recovering plastic materials from a post-consumer disposable absorbent article comprising a topsheet, a backsheet, an absorbent core, a hot melt adhesive H as defined herein, wherein at least one of the topsheet and the backsheet comprises at least two different plastic materials,
the method being characterized in that it comprises:
- a step i) of providing a post-consumer disposable absorbent article,
- a step ii) of immersing the post-consumer disposable absorbent article in an alkaline aqueous solution in conditions sufficient to obtain debonding of the at least two plastic materials,
- a step iii) of separation and recovering of the at least two different plastic materials from the medium, allowing the sorting and recovering of each individual plastic material in isolation.

### Additional steps

The method according to the invention may further comprise a rinsing step iv) of the plastic materials recovered after step iii).

The rinsing step iv) may last from 2 to 60 minutes, preferably from 5 to 50 minutes.

The rinsing step iv) may be carried out with water or with an organic solvent.

The amount of water or organic solvent used in step iv) may range from 500 to 5000 parts by mass with respect to 100 parts by mass of the plastic materials.

The method according to the invention may further comprise a dehydrating step v) of the plastic materials recovered after step iii) and/or step iv), when step iv) is performed with water.

The dehydrating step v) may be carried out using a dehydrator.

The dehydrating step v) may last from 1 to 15 minutes.

Preferably, the method as defined herein further comprises a step of drying vi) the plastic materials recovered at step iii), step iv) or step v).

The step of drying may be carried out using a dryer or a hot air drier.

The drying conditions may not be particularly restricted as long as they allow adequate drying of the recovered plastic material. For example, the drying temperature may range from 80°C to 200°C, more preferably from 100°C to 150°C. The drying time may range from 10 to 240 min.

The recovered plastic material (recovered either from step iii) or step iv) or step v)) is preferably submitted to a sterilization treatment.

The recovered plastic material is advantageously submitted to a pellet-forming step for reuse in plastic processing for example in injection molding.

The method of the present invention may further comprise a step of recovering superabsorbent polymers (SAP) from the medium, in case the post-consumer disposable absorbent article comprises superabsorbent polymers in the absorbent core. This step may be carried out with appropriate mechanical means such as for example by filtration. This optional step may be carried out after step iii).

The recovered superabsorbent polymers (SAP) may be subjected to additional steps of purification.

Preferably, in the method of the invention, the post-consumer disposable absorbent article is not submitted to ozone treatment and/or acidic treatment before step ii).

Preferably, the method of the invention does not comprise an extraction step with a solvent before step ii) (meaning no adding of solvent with the post-consumer disposable absorbent article).

Preferably, the method of the invention does not comprise a shredding step of the post-consumer disposable absorbent article before step ii) (it is not in fragments shape).

The method for recovering plastic material advantageously allows the recycling of recovered plastic material(s).

The method according to the invention advantageously allows the debonding, separation and recovering of high quality of plastic materials from a post-consumer disposable absorbent article. The method is advantageously easy to implement, efficient, economical, and smooth.

The present invention also relates to a method for recycling a post-consumer disposable absorbent article comprising a topsheet, a backsheet, an absorbent core, a hot melt adhesive H as defined herein, wherein at least one of the topsheet and the backsheet comprises at least one plastic material,
the method being characterized in that it comprises:
- a step i) of providing a post-consumer disposable absorbent article,
- a step ii) of immersing the post-consumer disposable absorbent article in an alkaline aqueous solution in conditions sufficient to obtain debonding of the plastic material(s),
- a step iii) of separation of the plastic material(s) from the medium into reusable raw material.

The definition, description, embodiments and preferred embodiments disclosed above for the post-consumer absorbent article, topsheet, backsheet, hot melt adhesive H, steps, in the method for recovering plastic material, apply for the method for recycling without needed to repeat them.

### B. Recycled/recovered plastic material

The present invention also relates to a recovered plastic material obtained by the method of recovering disclosed herein.

The recovered plastic material preferably contains less than 10 weight % of hot melt adhesive residue.

The recovered plastic material may be transformed into a film or pellet for advantageously subsequent reuse.

The ranges disclosed in this description include both the lower and the upper limit thereof. For example, the expressions "ranging from x to y" or "between x and y" comprises the limits x and y.

### Examples

The following examples illustrate the invention without limiting it.

### Description of raw materials:

The materials used were the following:
- Nyflex 223 (from NYNAS): a hydrotreated naphthenic process oil commercialized by Ergon (Acidic value and hydroxyl value below 1 mg KOH/g)
- Polyester A from VANDEPUTTE: a bio-based oligoester made by polycondensation from pure ricinoleic acid (Acidic value = 7 mg KOH/g and a hydroxyl value = 25 mg KOH/g)
- Crodabond CSA (from CRODA): bio-based oligoester made by polycondensation from castor oil and sebacid acid (Acidic value below 1 mg KOH/g and a hydroxyl value = 55 mgKOH/g)
- Escorez 5400 (from EXXON): hydrogenated hydrocarbon DCPD resin having a R&B softening point of 101°C (Acidic value and hydroxyl value below 1 mg KOH/g)
- Sylvalite 9000: a tall oil rosin ester commercialized by KRATON having a Ring and Ball softening point around 105°C and a renewable carbon content of 97%;
- Dercolyte^{®} M105: a polyterpene resin commercialized by DRT resulting from the polymerization of α-pinene and β-pinene and having a Ring and Ball softening point of 105°C
- Foral DX (from PINOVA) : hydrogenated rosin resin having a R&B softening point of 70°C (Acidic value below 158 mgKOH/g and hydroxyl value below 1 mgKOH/g)
- Irganox 1010: a phenolic type antioxidant commercialized by BASF;
- SINOPEC^{®} YH-1209 (from SINOPEC) is a linear SIS having 30% styrene and less than 1% diblock;
- SINOPEC^{®} YH-1126 (from SINOPEC) is a linear SIS copolymer having 16% styrene and 50% diblock content.

### Brookfield viscosity:

The Brookfield viscosity was measured according to the standard method ASTM D-3236, using a Brookfield viscometer of the type Spindle 27, at a temperature of about 149°C.

### Ring & Ball Softening Point

It is measured according to the standard method EN 1427, corresponding to the temperature at which a disk of material held in a ring and loaded with a ball will flow through a definite distance, when heated in glycerine.

### Acid value

It is given by supplier and determined by the standard method ASTM D1980.

### Hydroxyl value

It is given by supplier and determined by the standard method ASTM E1899-16.

### Example 1 : preparation of hot melt adhesives

The compositions of example 1 in Table 1 are prepared by simple mixing of its ingredients. Polymer(s) and antioxidant(s) are heated and melted at 150°C until everything is dissolved as a homogeneous mixture. Then, resin(s) are added at the same temperature until everything dissolved as a homogeneous mixture. At the end, the mixture is cooled down and collect to be used as it.

Compositions A, B, C (invention) and D (comparative) in the table below are compositions according to the invention.

**Table 1 : hot melt adhesive compositions**

| | Comparative composition D | A | B | C |
|---|---|---|---|---|
| Polyester A (%) | - | 16 | 8 | 16 |
| Nyflex 223 (%) | 22 | - | - | - |
| Crodabond CSA (%) | - | - | 8 | - |
| Escorez 5400 (%) | 57 | - | - | - |
| Dercolyte M105 (%) | - | 29.2 | 29.2 | - |
| Sylvalite 9000 (%) | - | 30.9 | 30.9 | 40.1 |
| Foral DX (%) | - | - | - | 20 |
| Sinopec 1209 (%) | 14 | 18 | 18 | 18 |
| Sinopec YH-1126 (%) | 6 | 4.6 | 4.6 | 4.6 |
| Irganox 1010 (%) | 1 | 1 | 1 | 1 |
| | 100 | 100 | 100 | 100 |
| Viscosity at 149°C (mPa.s) | 2500 | 3990 | 7920 | 3210 |
| R&B softening point (°C) | 85.0 | 88.3 | 99.4 | 73.4 |

### Example 2 : Preparation of a laminates by means of a slot nozzle coating equipment

A laminate was prepared as follows:
Use is made, as a laminating device, of a machine operating continuously at a line speed of approximately 200 m/minute, which machine is sold by NORDSON under the name of Coater CTL 4400.

In this machine, the coating nozzle is a slot nozzle, NORDSON SlotTM.

The two substrates employed are:
- a 22 g/m² PE film breathable which has a width of 20 cm, and
- a 15 g/m² spunbond hydrophile nonwoven sheet of the same width, which is composed of fibers of polypropylene (PP).

These two substrates are packaged as a roll with a width of 20 cm.

The hot melt adhesive compositions of table 1 were heated in the melting pot at a temperature of 150°C.

It was then coated at the same temperature of 150°C and at a coating weight of approximately 5 g/m2 on the PE film.

The resulting coating pattern is quite adequate and is typical of a good processability.

The nonwoven (PP) sheet was then put into contact with the coated surface of the PE film by means of a nip roll applying a pressure of 1 bar.

### Example 3 : performances

The laminates obtained in example 2 were then packaged as a roll and left for 24 hours at ambient temperature (23°C) and at 50% relative humidity.

A rectangular strip measuring 2.54 cm by approximately 10 cm was then cut out in the coated central area of the laminate.

### Dry peel measurement

The two individual substrates were separated, starting from one end of the above rectangular strip (as a test specimen) and over approximately 2 cm.

The two free ends thus obtained were fixed to two clamping devices respectively connected to a stationary part and a movable part of a tensile testing device which are located on a vertical axis.

While a drive mechanism communicates, to the movable part, a uniform speed of 300 mm/minute, resulting in the separation of the two substrates, the separated ends of which are gradually displaced along a vertical axis while forming an angle of 180°, the stationary part, connected to a dynamometer, measures the force withstood by the test specimen thus held.

The result corresponding to the peel after 24 hours at 23°C, is expressed in N/25 mm.

The results are reported in Table 2 below.

### Wet peel measurement

The rectangular strip is totally immerged in a deionized water bath at 30°C for 30 seconds and then removed and dried. The same protocol as "dry peel measurement" is applied to determine the peel strength after wet conditions treatment.

The results are also reported in Table 2 below.

### Treatment method (washing alkaline solution)

The rectangular strip was totally immerged in a 2wt% NaOH solution bath at 30°C for 30 seconds (under mixing : 300 rpm), and then was removed with a clamp, and dried.

The same protocol as "dry peel measurement" was applied to determine the peel strength after the treatment method (NaOH).

The results are reported in table 2 below.

**Table 2 : performances**

| | Comparative composition D | A (invention) | B (invention) | C (invention) |
|---|---|---|---|---|
| Initial dry Peel (N/25 mm) | 2.8 | 3.1 | 2.7 | 3.1 |
| Initial Wet peel (N/25 mm) | 3.1 | 3.0 | 2.8 | 2.8 |
| Dry peel after treatment (N/25 mm) | 3.2 | 0.1 | 0.4 | 0.0 |
| Difference between initial dry peel and dry peel after treatment (N/25 mm) (%) | +12% | -97% | -85% | -100% |

The table demonstrates that the use the hot melt adhesive compositions A, B, C (according to invention) advantageously allows the debonding of two substrates mentioned above. Indeed, the dry peel after treatment of the invention is advantageously low even egal to zero. There is a loss of adhesion (dry peel) of at least 85% for compositions B and C, and more than 95% with compositions A and C, after only 30 seconds of treatment. This is not the case with comparative example D where there is no reducing of the dry peel after the treatment of the invention.

## Claims

1. Method for recovering plastic material(s) from a post-consumer disposable absorbent article comprising a topsheet, a backsheet, an absorbent core, a hot melt adhesive composition H, wherein at least one of the topsheet and the backsheet comprises at least one plastic material,
wherein the hot melt adhesive composition H comprises:
∘ at least one polymer P selected from the group consisting of polyolefins, thermoplastic block copolymers of a vinyl-based aromatic hydrocarbon and a conjugated diene compound, and mixtures thereof;
∘ at least one tackifier T comprising at least one ester group or carboxylic acid group;
the method being **characterized in that** it comprises:
- a step i) of providing a post-consumer disposable absorbent article,
- a step ii) of immersing the post-consumer disposable absorbent article in an alkaline aqueous solution in conditions sufficient to obtain debonding of plastic material(s),
- a step iii) of separation and recovering of plastic material(s) from the medium.

2. Method according to claim 1, wherein the plastic material is selected from the group consisting of: polyolefins (such as for example polyethylene, polypropylene, ...), polyesters (such as for example polyethylene terephthalate, polybutylene terephthalate), polyamides (such as for example 6-nylon, 6,6-nylon), poly(lactic acid), and mixtures thereof.

3. Method according to anyone of claims 1 to 2, wherein the topsheet comprises a non-woven material, said non-woven material preferably comprising plastic material, even more preferably the topsheet comprises a non-woven material comprising polypropylene.

4. Method according to anyone of claims 1 to 3, wherein the backsheet comprises a plastic film comprising a plastic material, and more particularly a plastic material selected from polyethylenes.

5. Method according to anyone of claims 1 to 4, wherein the hot melt adhesive composition H comprises one or several thermoplastic block copolymers of a vinyl-based aromatic hydrocarbon and a conjugated diene compound.

6. Method according to anyone of claims 1 to 5, wherein the thermoplastic block copolymer is chosen from the group consisting of:
- styrene-butadiene-styrene copolymer (SBS),
- styrene-butadiene diblock (SB),
- styrene-isoprene-styrene copolymer (SIS),
- styrene-isoprene diblock (SI),
- styrene-ethylene-butylene-styrene copolymer (SEBS),
- styrene-propylene-butylene-styrene copolymer (SPBS),
- styrene-butadiene-butylene-styrene copolymer (SBBS),
- styrene-ethylene-propylene-styrene copolymer (SEPS),
- styrene-ethylene-ethylene-propylene-styrene copolymer (SEEPS),
- styrene-isoprene-butadiene-styrene copolymer (SIBS),
- styrene-isoprene-propylene-styrene copolymer (SIPS),
- styrene-farnesene copolymer (SFC),
- and any mixture thereof.

7. Method according to anyone of claims 1 to 6, wherein the hot melt adhesive composition H comprises at least one plasticizer, preferably selected from the group consisting of naphthenic and paraffinic oils, liquid olefin polymers, plasticizers comprising at least one ester group or carboxylic acid group, and mixtures thereof, even more preferably selected from fatty acid oligomers/polymers.

8. Method according to anyone of claims 1 to 7, wherein the hot melt adhesive composition H exhibits an average initial dry peel strength higher than 2.0 N/25 mm, more preferably higher than 2.0 N/25 mm, even more preferably higher than 2.5 N/25 mm with an adhesive coating weight of 5 g/m².

9. Method according to anyone of claims 1 to 8, wherein the hot melt adhesive composition H exhibits an average initial wet peel strength higher than 2.0 N/25 mm, more preferably higher than 2.0 N/25 mm, even more preferably higher than 2.5 N/25 mm with an adhesive coating weight of 5 g/m².

10. Method according to anyone of claims 1 to 9, wherein in step ii), the alkaline aqueous solution is an aqueous solution of sodium hydroxide, potassium hydroxide, lithium hydroxide, and mixtures thereof, preferably the alkaline aqueous solution is an aqueous solution of sodium hydroxide.

11. Method according to anyone of claims 1 to 10, wherein in step ii), the concentration of hydroxide ions in the alkaline solution ranges from 1 wt% to 10 wt%, preferably from 1 wt% to 5 w%.

12. Method according to anyone of claims 1 to 11, wherein in step ii), the pH of the alkaline aqueous solution ranges from 8 to 14, more preferably from 10 to 14.

13. Method according to anyone of claims 1 to 12, wherein in step ii), the conditions sufficient to obtain debonding of plastic material(s) are:
- at a temperature lower than the softening temperature of the hot melt adhesive composition H,
- for a time ranging from 10 second to 24 hours , preferably 10 second to 6 hours

14. Method according to anyone of claims 1 to 13, wherein at least one of the topsheet and the backsheet comprises at least two different plastic materials, the method being **characterized in that** it preferably comprises:
- a step i) of providing a post-consumer disposable absorbent article,
- a step ii) of immersing the post-consumer disposable absorbent article in an alkaline aqueous solution in conditions sufficient to obtain debonding of the at least two plastic materials,
- a step iii) of separation and recovering of the at least two different plastic materials from the medium, allowing the sorting and recovering of each individual plastic material in isolation.

15. Method according to anyone of claims 1 to 14, wherein the post-consumer disposable absorbent article is not submitted to ozone treatment and/or acidic treatment before step ii).

16. Method according to anyone of claims 1 to 15, **characterized in that** it does not comprise a shredding step of the post-consumer disposable absorbent article before step ii).
